# EUROPEAN PATENT APPLICATION

(11) **EP 2 644 098 A1**
(43) Date of publication of application: **02.10.2013**
(21) Application number: 11843107.1
(22) Date of filing: 22.04.2011
(51) Int. Cl.: A61B 8/00, G01N 29/24

(54) **HIGH-OUTPUT ULTRASOUND PROBE**

(30) Priority: 22.11.2010 KR 20100116419
(71) Applicant: GE Healthcare Co., Ltd., Seongnam-si, Gyeonggi-do 462-120 (KR); Humanscan Co., Ltd., Ansan-si, Gyeonggi-do 425-110 (KR)
(72) Inventor: KIM, Jeong Seok, Seoul 140-731 (KR); RHIM, Sung Min, Incheon 405-300 (KR); JUNG, Ho, Seoul 151-050 (KR); OH, Duck Hwan, Suwon-si Gyeonggi-do 443-470 (KR)
(74) Representative: Gassner, Wolfgang
(86) International application number: PCT/KR2011/002908
(87) International publication number: WO 2012/070731

(57) **Abstract**

Provided is an ultrasonic probe, which uses a composite piezo-electric element, which includes a piezo-electric wafer made of piezo-electric material and having a lattice shape, and a polymer filled between the lattices of the piezoelectric wafer. The ultrasonic probe has a high power and a wide band width simultaneously. In addition, since the ultrasonic probe further includes a heat exhaust unit for exhausting the heat generated at the ultrasonic probe to the outside, the temperature of an acoustic lens which contacts patients may be lowered.

## Description

### [Technical Field]

The present disclosure relates to an ultrasonic probe, and more particularly, to an ultrasonic probe having a high power capable of inducing vibration of the human anatomy and allowing the imaging diagnosis.

### [Background Art]

In a general ultrasonic imaging diagnosis, ultrasonic waves are transferred to a human body and then an image is obtained from signals reflected from the human body and utilized for diagnosis. However, in these days, the trend is not only to simply realize an image with reflection signals but also to obtain a lot of information by using various changes of the human anatomy which occurs when ultrasonic waves are transferred to a human body and utilize such information for diagnosis.

Among such information, the vibration information of the human anatomy caused by ultrasonic waves transferred to the human body is utilized for diagnosis more and more. For this, high-power ultrasonic waves capable of causing vibration of a human body should be used. Therefore, an ultrasonic probe used in such applications should have a high power and a wide band width which does not cause any problem in the imaging diagnosis. In other words, the ultrasonic probe used for utilizing the vibration information of the human anatomy caused by the ultrasonic waves transferred to a human body should have a high power and a wide band width, but implementing such an ultrasonic probe is very difficult.

### [Disclosure]

### [Technical Problem]

A first object of the present disclosure is to provide a method for producing a high-power composite wafer having a high power and a wide band width simultaneously.

A second object of the present disclosure is to provide a high-power composite wafer produced by the above high-power composite wafer producing method, which has a high power and a wide band width simultaneously.

A third object of the present disclosure is to provide a composite piezo-electric element, which has a high power and a wide band width simultaneously.

A fourth object of the present disclosure is to provide an ultrasonic probe, which has a high power and a wide band width simultaneously.

### [Technical Solution]

In order to accomplish the first object aspect, the present disclosure provides a method for producing a high-power composite wafer, which includes: cutting a wafer made of piezo-electric material into a lattice shape; filling a polymer between the cut lattices of the wafer; lapping the wafer; and generating an electrode at the wafer.

In order to accomplish the second object aspect, the present disclosure provides a high-power composite wafer, produced by the high-power composite wafer producing method.

The high-power composite wafer according to another embodiment of the present disclosure may include: a piezo-electric wafer made of piezo-electric material and having a lattice shape; and a polymer filled between the lattices of the piezo-electric wafer.

In order to accomplish the third object aspect, the present disclosure provides a composite piezo-electric element, which uses the high-power composite wafer produced by the high-power composite wafer method.

The composite piezo-electric element according to another embodiment of the present disclosure may use a composite wafer which includes a piezo-electric wafer made of piezo-electric material and having a lattice shape; and a polymer filled between the lattices of the piezo-electric wafer.

In order to accomplish the fourth object aspect, the present disclosure provides an ultrasonic probe, which includes the composite piezo-electric element using the high-power composite wafer produced by the high-power composite wafer method.

According to an embodiment of the present disclosure, the ultrasonic probe may further include: an acoustic lens for focusing or diffusing ultrasonic waves; and a heat exhaust unit connected to the acoustic lens to emit heat generated by the acoustic lens.

### [Advantageous Effects]

According to the present disclosure, an ultrasonic probe having a high power and a wide band width simultaneously may be provided. In addition, according to the present disclosure, since the heat generated at the ultrasonic probe with a high power is exhausted to the outside, the temperature of an acoustic lens which contacts patients may be lowered.

### [Description of Drawings]

Fig. 1 shows a structure of an ultrasonic probe which generates low-power ultrasonic waves to obtain an ultrasonic image.
Fig. 2 shows a structure of an ultrasonic probe which generates high-power ultrasonic waves to obtain an ultrasonic image.
Fig. 3 shows a structure of a high-power ultrasonic probe according to a preferred embodiment of the present disclosure.
Fig. 4 shows acoustic simulation results of a general wafer and a composite wafer having a high Q value according to an embodiment of the present disclosure.
Fig. 5 is a flowchart for illustrating a method for producing a composite wafer having a high Q value according to an embodiment of the present disclosure.
Fig. 6 shows a wafer at each stage in the method for producing a composite wafer having a high Q value.
Fig. 7 shows an ultrasonic probe using a composite wafer manufactured according to another embodiment of the present disclosure.

### [Best Mode]

A method for producing a high-power composite wafer according to an embodiment of the present disclosure includes cutting a wafer made of piezo-electric material into a lattice shape, filling a polymer between the cut lattices of the wafer, lapping the wafer, and generating an electrode at the wafer.

### [Mode for Invention]

Hereinafter, the present disclosure will be described in more detail based on preferred embodiments. However, these embodiments are for better understanding of the present disclosure, and it is obvious to those skilled in the art that the scope of the present disclosure is not limited thereto. In order to clarify the solution of the present disclosure to the problems, the configuration of the present disclosure is described in detail with reference to the accompanying drawings where the same component is endowed with the same reference symbol at different drawings. Moreover, when a drawing is explained, a component depicted in another drawing may be recited. In addition, in a case where detailed description of known functions or configurations in relation to the present disclosure is judged as unnecessarily making the essence of the present disclosure vague, the detailed description will be excluded.

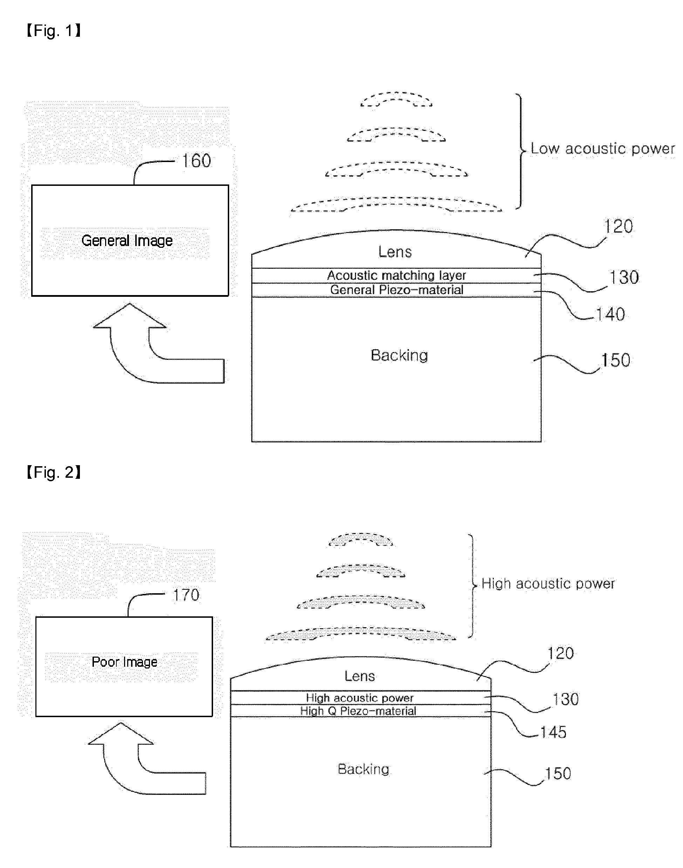

Fig. 1 shows a structure of an ultrasonic probe which generates low-power ultrasonic waves to obtain an ultrasonic image.

Referring to Fig. 1, the ultrasonic probe includes a lens 120, an acoustic matching layer 130, a piezo-electric element 140 and a back side layer 150.

The lens 120 allows ultrasonic waves to progress with time differences in order to focus the ultrasonic waves.

The acoustic matching layer 130 is located at the front surface of the piezo-electric element 140 and serves as a layer which allows ultrasonic waves to be easily transferred between the piezo-electric element 140 and an article. The acoustic matching layer 130 matches acoustic impedance to the article.

The piezo-electric element 140 is an active element for generating and receiving ultrasonic waves and generally uses piezo-electric material as an active element. The sensitivity and resolution of an ultrasonic wave sensor have close relation with electrical and mechanical characteristics of the piezo-electric element. The piezo-electric material uses Plumbum-Zironate-Titanate (PZT) piezo-electric ceramic having a great electro-mechanical coupling factor, without being limited thereto. However, the piezo-electric ceramic has very large acoustic impedance and thus is suffered from impedance mismatching. The acoustic impedance mismatching is resolved by using the acoustic matching layer 130 between the piezo-electric element 140 and the article.

The back side layer 150 is located at the rear surface of the piezo-electric element 140 and absorbs ultrasonic waves irradiated backwards to reduce the length of ultrasonic pulses. As the acoustic impedance of the back side layer 150 is closer to the acoustic impedance of the piezo-electric element, less reflection occurs at their border surface to reduce a duration period, which improves an axial resolution and, in an ideal case, allows very short positive ultrasonic pulses to be obtained. However, since the sensitivity of the ultrasonic wave sensor deteriorates as the sound absorption loss by the back side layer 150 increases, there needs a compromise with a pulse length. The back side layer 150 may be made of material obtained by mixing epoxy with tungsten and purifying the same.

The ultrasonic probe shown in Fig. 1 should have a wide band width in order to obtain a clear image, and for this, piezo-electric material having a high electro-mechanical coupling factor is used. The piezo-electric material having a high electro-mechanical coupling factor generally has a low Q value and so is unable to give a high power.

Generally, the ultrasonic probe is not easily used as a probe having a high acoustic power and allowing imaging diagnosis simultaneously. Since a general ultrasonic probe uses a piezo-electric element having a low Q value, which is suitable for imaging diagnosis, there is a limit in applicable voltages, and thus it is impossible to apply a high power and output a high acoustic power.

Fig. 2 shows a structure of an ultrasonic probe which generates high-power ultrasonic waves to obtain an ultrasonic image.

The ultrasonic probe shown in Fig. 2 may be configured in the same way as the ultrasonic probe shown in Fig 1, except for the piezo-electric element 140.

While the piezo-electric element 140 shown in Fig. 1 uses piezo-electric material having a low Q value, the piezo-electric element 145 shown in Fig. 2 uses piezo-electric material having a high Q value.

The piezo-electric material having a high Q value is used to output a desired high power. In this case, although a high acoustic power may be output, an image having a desired quality may not be obtained. In a case where piezo-electric material having a high Q value is used, an acoustic power may be enhanced, but a signal of a wide band width may not be easily obtained.

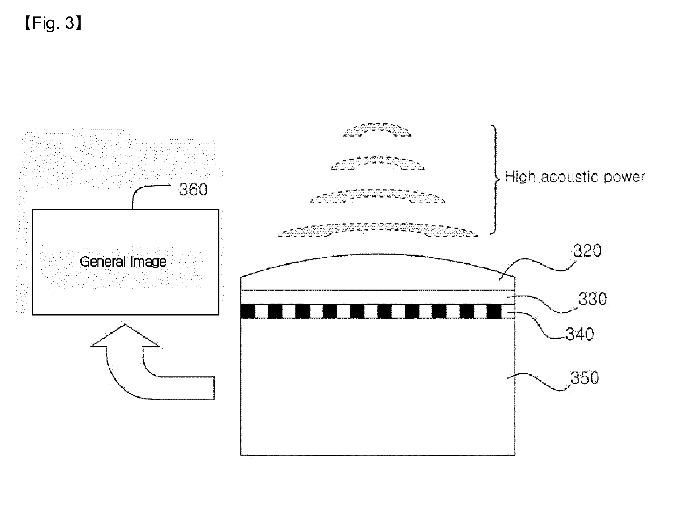

Fig. 3 shows a structure of a high-power ultrasonic probe according to a preferred embodiment of the present disclosure.

Referring to Fig. 3, the high-power ultrasonic probe of this embodiment includes an acoustic lens 320, an acoustic matching layer 330, a piezo-electric element 340 and a back side layer 350.

The acoustic lens 320, the acoustic matching layer 330 and the back side layer 350, shown in Fig. 3, may be identical to the lens 120, the acoustic matching layer 130 and the back side layer 150, shown in Fig. 1.

Now, differences between the piezo-electric element 340 shown in Fig. 3 and the piezo-electric elements 140, 145 shown in Figs. 1 and 2 will be described.

The piezo-electric element 340 according to an embodiment of the present disclosure uses piezo-electric material having a high Q value, which is produced in the form of a composite material with a polymer. The piezo-electric material having a high Q value allows a high voltage to be applied, and the composite material with a polymer enhances conversion efficiency. Therefore, the piezo-electric element 340 according to an embodiment of the present disclosure may output a high acoustic power and obtain a signal of a wide band width, simultaneously.

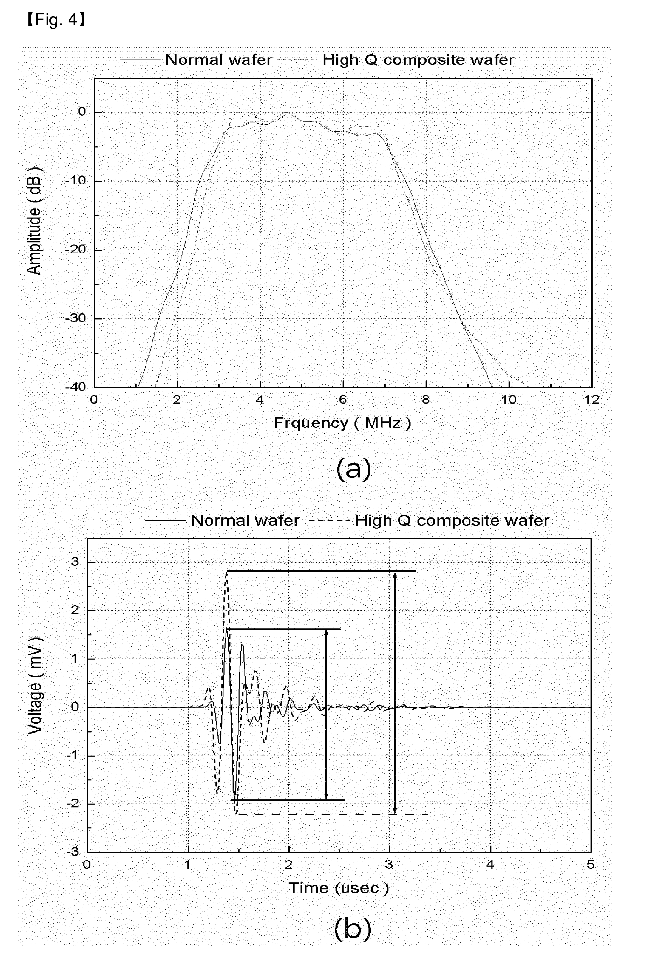

Fig. 4 shows acoustic simulation results of a general wafer and a composite wafer having a high Q value according to an embodiment of the present disclosure.

A portion (a) of Fig. 4 shows a FFT spectrum result and a portion (b) of Fig. 4 shows a pulse-echo wave.

If the case where a probe is made using a general piezo-electric wafer and the case where a probe is made using a composite wafer having a high Q value are simulated, it could be understood that the composite wafer has high sensitivity or power while maintaining an existing band width.

**[Table 1]**

| | Sensitivity | -6dB | | | |
|---|---|---|---|---|---|
| | | F1 | F2 | CF | BW |
| Dim. | dB | MHz | MHz | MHz | % |
| Normal | 0 | 2.89 | 7.17 | 5.03 | 85 |
| High Q comp. | -4.2 | 3.05 | 7.14 | 5.10 | 83 |

Referring to Fig. 4 and Table 1, even though the band width is not greatly different, it could be found that the sensitivity of signal of the probe manufactured by using the composite wafer is greater than that of an existing product by about 4 dB or more. In addition, since the material having a high Q value is used, a higher voltage may be applied in comparison to an existing product, and so its acoustic power would be further enhanced.

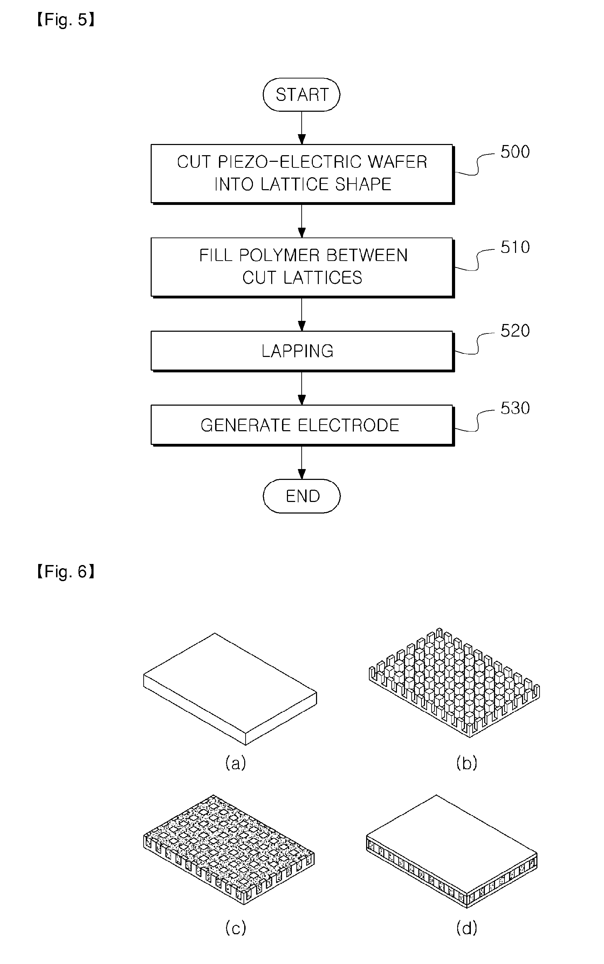

Fig. 5 is a flowchart for illustrating a method for producing a composite wafer having a high Q value according to an embodiment of the present disclosure, and Fig. 6 shows a wafer at each stage in the method for producing a composite wafer having a high Q value.

Hereinafter, the method for producing a composite wafer having a high Q value according to the present disclosure will be described in detail with reference to Figs. 5 and 6.

In Operation 500, a piezo-electric wafer is cut into a lattice shape. Referring to portions (a) and (b) of Fig. 6, it could be understood that an original piezo-electric wafer (the portion (a) of Fig. 6) is changed into a wafer (the portion (b) of Fig. 6) cut into a lattice shape.

In Operation 510, a polymer is filled between the cut lattices. Referring to a portion (c) of Fig. 6, it could be understood that a polymer is filled between the cut lattices. The polymer may use epoxy.

In Operation 520, the wafer is lapped to become flat. The wafer is pressed on a plate with good flatness, and then the wafer and the plate are moved relatively while applying an abrasive thereto, thereby removing fragments of the wafer. The abrasive may use a mixture of Al₂O₃ and glycerin.

In Operation 530, an electrode is formed at one surface of the wafer to make a composite wafer. A portion (d) of Fig. 6 shows a composite wafer where an electrode is formed. Seeing the section of the composite, it could be understood that piezo-electric material and polymer are located alternately.

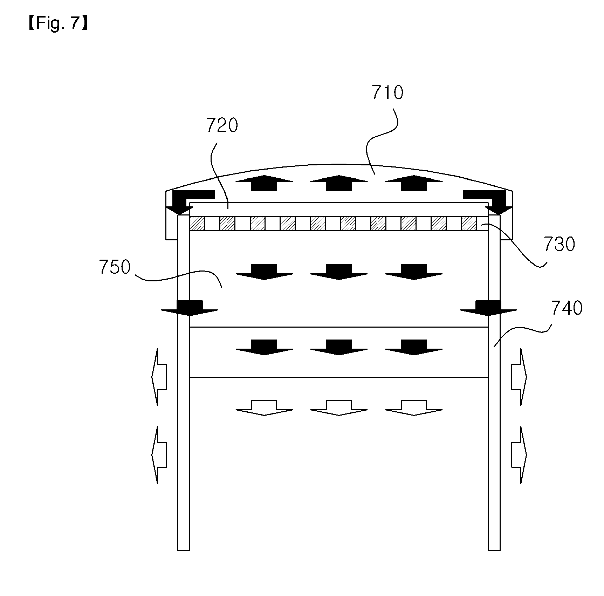

Fig. 7 shows an ultrasonic probe using a composite wafer manufactured according to another embodiment of the present disclosure.

Referring to Fig. 7, the ultrasonic probe of this embodiment includes an acoustic lens 710, an acoustic matching layer 720, a composite piezo-electric element 730, a heat exhaust unit 740 and a back side layer 750.

The acoustic lens 710 allows ultrasonic waves to progress with time differences in order to focus or diffuse the ultrasonic waves.

The acoustic matching layer 720 is located at the front surface of the composite piezo-electric element 730 and serves as a layer which allows ultrasonic waves to be easily transferred between the composite piezo-electric element 730 and an article. The acoustic matching layer 720 matches acoustic impedance to the article.

The composite piezo-electric element 730 is configured so that piezo-electric material and polymer are alternately arranged as shown in Fig. 5 and gives a high acoustic power with a high Q value.

The heat exhaust unit 740 is an exhaust passage for exhausting heat of the acoustic lens 710. Since the ultrasonic probe of this embodiment has a high acoustic power, a lot of heat is generated by the composite piezo-electric element 730 which vibrates, and this heat may give harm to patients. Therefore, the temperature near the acoustic lens 710 which contacts patients should be limited.

However, if a high acoustic power is not useable due to the limit of the surface temperature of the acoustic lens 710 even though the composite piezo-electric element 730 is designed to give a high acoustic power, this will become a problem. Therefore, it is very important to satisfactorily exhaust the heat generated at the composite piezo-electric element 730 to the outside. The heat exhaust unit 740 is preferably connected to the acoustic lens 710 to exhaust the heat of the acoustic lens 710 to the outside. However, the heat exhaust unit 740 may also be connected to the composite piezo-electric element 730 or the acoustic matching layer 720 to exhaust heat to the outside.

The back side layer 750 is located at the rear surface of the composite piezo-electric element 730 and absorbs ultrasonic waves irradiated backwards, thereby reducing the length of ultrasonic pulses. As the acoustic impedance of the back side layer 750 is closer to the acoustic impedance of the piezo-electric element 730, less reflection occurs at their border surface to reduce a duration period, which improves an axial resolution and, in an ideal case, allows very short positive ultrasonic pulses to be obtained. However, since the sensitivity of the ultrasonic wave sensor deteriorates as the sound absorption loss by the back side layer 750 increases, there needs a compromise with a pulse length. The back side layer 750 may be made of material obtained by mixing epoxy with tungsten and purifying the same.

The present disclosure has been described based on various embodiments. A person having ordinary skill in the art will understand that the present disclosure may be modified without departing from the spirit of the present disclosure. Therefore, the disclosed embodiments should be interpreted not in a limiting aspect but in a descriptive aspect. The scope of the present disclosure is not defined by the above description but by the appended claims, and all differences equivalent to the present disclosure should be interpreted to be included in the present disclosure.

## Claims

1. A method for producing a high-power composite wafer, comprising:
cutting a wafer made of piezo-electric material into a lattice shape;
filling a polymer between the cut lattices of the wafer;
lapping the wafer; and
generating an electrode at the wafer.

2. A high-power composite wafer, produced by the method defined in the claim 1.

3. A composite piezo-electric element, which uses the high-power composite wafer produced by the method defined in the claim 1.

4. An ultrasonic probe, which includes the composite piezo-electric element using the high-power composite wafer produced by the method defined in the claim 1.

5. The ultrasonic probe according to claim 4, further comprising:
an acoustic lens for focusing or diffusing ultrasonic waves; and
a heat exhaust unit connected to the acoustic lens to emit heat generated by the acoustic lens.

6. A composite wafer, comprising:
a piezo-electric wafer made of piezo-electric material and having a lattice shape; and
a polymer filled between the lattices of the piezo-electric wafer.

7. A composite piezo-electric element, which uses a composite wafer including:
a piezo-electric wafer made of piezo-electric material and having a lattice shape; and
a polymer filled between the lattices of the piezo-electric wafer.

8. An ultrasonic probe, comprising the composite piezo-electric element defined in the claim 5.

9. The ultrasonic probe according to claim 8, further comprising:
an acoustic lens for focusing or diffusing ultrasonic waves; and
a heat exhaust unit connected to the acoustic lens to emit heat generated by the acoustic lens.
